# FASCICULE DE BREVET EUROPEEN

(11) **EP 0 647 608 B1**
(45) Date de publication et mention de la délivrance du brevet: **23.12.1998**
(21) Numéro de dépôt: 94402163.3
(22) Date de dépôt: 28.09.1994
(51) Int. Cl.: C07C 41/06, C07C 41/42, C07C 43/04

(54) **Procédé de production d'éthers tertiaires à partir d'une charge C4 ou C5 comprenant deux étapes de distillation extractive**
Verfahren zur Herstellung von tertiären Ethern aus C4 oder C5 Rohstoffen, das extraktive Distillation enthält
Process of production of tertiary ethers from C4 or C5 feedstock, including extractive distillation

(30) Priorité: 08.10.1993 FR 9312103
(43) Date de publication de la demande: 12.04.1995
(73) Titulaire: INSTITUT FRANCAIS DU PETROLE, 92502 Rueil-Malmaison (FR)
(72) Inventeur: Minkkinen, Ari, F-78860 Saint Nom la Breteche (FR); Mikitenko, Paul, F-78590 Noisy le Roy (FR); Asselineau, Lionel, F-75017 Paris (FR)

(56) Documents cités:
- FR-A- 2 520 356
- FR-A- 2 527 201
- FR-A- 2 527 202
- FR-A- 2 614 297
- GB-A- 2 068 408
- CHEMICAL ECONOMY AND ENGINEERING REVIEW, vol.14, no.6, Juin 1982 pages 35 - 40 G MARCEGLIA ET AL 'SNAMPROGETTI INTEGRATED ISOMERIZATON/MTBE PRODUCTION BLOCK FLOW DIAGRAM'

## Description

L'invention concerne un procédé de production d'éthers tertiaires (méthyltertiobutyléther : MTBE, et éthyltertiobutyléther : ETBE, méthyltertioamylether : TAME et ethyltertioamylether : ETAE) à partir d'une coupe hydrocarbonée comportant des hydrocarbures à quatre ou à cinq atomes de carbone, partiellement oléfiniques.

Ces éthers sont introduits dans les essences pour augmenter l'indice d'octane et on estime à l'heure actuelle que la demande mondiale en MTBE, par exemple, croît de 25 % par an. Pour répondre à cette demande, les isooléfines à carbone tertiaire aisément disponibles à partir des unités de production d'oléfines existantes et des craqueurs catalytiques en lit fluidisé ne suffisent pas. On doit prendre en compte la totalité des oléfines disponibles afin de les isomériser sous forme d'oléfines tertiaires qui est la forme réactive et de satisfaire ainsi la demande future en éthers tertiaires.

Le développement de la technologie d'isomérisation squelettale rend possible aujourd'hui la réalisation de cet objectif. La combinaison de la synthèse d'éthers tertiaires par réaction d'isooléfines tertiaires et de méthanol ou d'éthanol, et de l'isomérisation squelettale des oléfines qui ne réagissent pas ou très peu en étherification mérite donc aujourd'hui toute l'attention des industriels.

L'art antérieur est illustré par les brevets suivants : FR-A-2 527 202, FR-A-2 614 297, FR-A-2 520 356, GB-A-2 068 408, et par la publication Chemical economy and engineering review vol 14, N°6, Juin 1982, pages 35-40 (Integrated isomerization/MTBE production block flow diagram)

Le brevet FR-A 2 527 201 décrit un procédé de synthèse d'éthers tertiaires où l'effluent produit contenant notamment des hydrocarbures saturés et oléfiniques subit une distillation extractive pour séparer les hydrocarbures saturés des hydrocarbures oléfiniques. La présence d'alcool non converti dans ledit effluent n'est pas mentionnée, ce qui peut laisser supposer que la réaction est réalisée dans des conditions stoechiométriques et donc avec une conversion qui n'est pas optimisée.

Il est connu que les technologies d'isomérisation squelettale ne sont pas sans contraintes physiques et thermodynamiques. En effet la conversion n'atteint que 30 à 50 % avec une sélectivité de 75 à 85 %, ce qui nécessite des recyclages substantiels pour convertir toutes les oléfines en isooléfines tertiaires. De plus, pour atteindre les performances citées, le procédé doit être opéré à des pressions plutôt faibles, ce qui nécessite de gros équipements pour manipuler les volumes d'effluents qui, à leur tour, doivent être comprimés en vue de la séparation et de la récupération des produits.

Un autre obstacle à une combinaison efficace de l'isomérisation squelettale et de la synthèse d'éthers tertiaires résulte du fait que c'est seulement la fraction oléfinique qui est convertie dans l'isomérisation squelettale. Les hydrocarbures saturés, en effet, traversent le réacteur d'isomérisation comme des composés inertes et sont récupérés avec les composés isooléfiniques produits, ce qui contribue à diluer les hydrocarbures réactifs de manière permanente.

Les charges hydrocarbonées en C₄ ou en C₅ provenant des unités de production d'oléfines et des craqueurs catalytiques sont très variables en raison aussi bien de leur teneur en isooléfines que de la nature même des oléfines présentes. De ce fait, les effluents de synthèse d'éthers tertiaires sont très différents et les schémas de raffinage en aval doivent être ajustés en conséquence. Par exemple, les effluents d'unités de production d'oléfines sont riches en oléfines linéaires et pauvres en hydrocarbures saturés, et à ce titre sont considérés comme de bonnes charges d'isomérisation. Par contre, les effluents en C₄ et en C₅ de craquage catalytique contiennent en général plus de 50 % d'hydrocarbures saturés et nécessitent donc en aval des équipements de très grande taille qui par ailleurs consomment énormément d'énergie.

De plus, quelle que soit la qualité de ces effluents, le produit d'isomérisation squelettale ne peut pas être recyclé en amont du réacteur de synthèse d'éthers tertiaires sans une purge substantielle des hydrocarbures saturés accumulés, notamment dans le cas de charges de craquage catalytique. Cette étape de purge devient donc un obstacle majeur dans le procédé de synthèse. Un des objets de l'invention est de remédier aux inconvénients ou limitations mentionnées ci-avant, et notamment d'éliminer avant l'étape d'isomérisation au moins une partie des hydrocarbures saturés présents dans la charge.

On a trouvé selon l'invention qu'en combinant une étape de distillation extractive des oléfines par un solvant sélectif des oléfines entre l'étape de synthèse d'éthers tertiaires et l'étape d'isomérisation squelettale des oléfines non converties, on obtenait de très bons résultats.

De manière détaillée, l'invention propose un procédé de production d'éthers tertiaires dans lequel on réalise les étapes suivantes :
a) On fait réagir dans des conditions de synthèse appropriées une charge hydrocarbonée contenant des hydrocarbures saturés et des hydrocarbures isooléfiniques tertiaires et notamment de l'isobutène ou du 2- méthyl 2-butène et 2-méthyl 1-butène avec un alcool en excés qui est le méthanol si l'on produit du méthyltertiobutyléther (MTBE) ou du méthyltertioamyléther (TAME), ou l'éthanol si l'on produit de l'éthyltertiobutyléther (ETBE) ou de l'éthyltertioamyléther (ETAE) dans au moins une zone de réaction catalytique et on produit un effluent contenant l'éther tertiaire.
b) On distille l'effluent dans une zone de distillation (C_{I}) dans des conditions de distillation adéquates et on récupère, en fond de zone de distillation l'éther tertiaire, et en tête un distillat comprenant des hydrocarbures oléfiniques, une partie au moins dudit alcool et les hydrocarbures saturés, que, éventuellement, l'on condense au moins en partie .
c) On réalise dans une première zone de distillation extractive (C_{II}), dans des conditions de première distillation extractive appropriées, une distillation extractive du distillat de l'étape b), en présence d'un solvant non aqueux sélectif des hydrocarbures oléfiniques qui est introduit au moins en partie dans ladite première zone et on récupère une fraction de tête riche en au moins une partie des hydrocarbures saturés que l'on condense au moins en partie et une fraction de fond de première distillation extractive.
d) On réalise dans une seconde zone de distillation extractive (C_{III}), dans des conditions de seconde distillation extractive adéquates une distillation extractive de la fraction de fond de l'étape c) et l'on récupère en tête au moins en partie un distillat de seconde distillation extractive riche en hydrocarbures oléfiniques que l'on condense au moins en partie et en fond, un résidu contenant essentiellement de l'alcool et du solvant.
e) On effectue une étape de séparation du résidu de l'étape d), par exemple par strippage, dans une zone de séparation (C_{IV}), dans des conditions de séparation appropriées et l'on récupère en tête une partie au moins de l'alcool que l'on recycle au moins en partie à l'étape a) et en fond du solvant que l'on recycle au moins en partie à l'étape c).
f) On réalise une isomérisation squelettale du distillat de l'étape d) dans une zone d'isomérisation squelettale comprenant au moins une zone de séparation des effluents d'isomérisation, dans des conditions d'isomérisation squelettale appropriées et l'on récupère en provenance de la zone de séparation desdits effluents, un premier distillat d'isomérisation contenant des hydrocarbures légers et un deuxième distillat d'isomérisation contenant les hydrocarbures isooléfiniques tertiaires que l'on recycle à l'étape a).

Dans le cas d'une charge correspondant à une coupe C₄, la zone de séparation des effluents d'isomérisation comprend en général un dépropaniseur et un débutaniseur.

Dans le cas d'une coupe C₅, elle comprend en général un débutaniseur et un dépentaniseur.

Il est bien entendu que lorsqu'on produit du MTBE ou du TAME, l'alcool mis en jeu dans toutes les étapes du procédé est le méthanol. De la même façon, lorsqu'on produit de l'ETBE ou de l'ETAE, l'alcool mis en jeu est l'éthanol.

Le solvant mis en jeu est un solvant ayant une substantielle sélectivité vis à vis des oléfines. Il peut être séparé des alcools par strippage ou par distillation, il peut être séparé des constituants de la charge et il est généralement miscible avec les constituants de la charge.

Parmi les solvants répondant à ces définitions, on a observé que la N-N diméthylformamide, la N-N diéthylformamide, la N-N diméthylacétamide, la formylmorpholine, la N-méthylpyrrolidone, la butyrolactone et le furfural donnaient de bons résultats et plus particulièrement le N-N diméthylformamide.

On opère avantageusement dans la seconde zone de distillation extractive à une température de fond de colonne, n'excédant pas en général 220°C et choisie en fonction du solvant pour éviter sa décomposition.

Les avantages du procédé par rapport à ceux de l'art antérieur sont les suivants :

On peut récupérer l'alcool en excès qui distille par azéotropie avec l'effluent brut de tête de colonne de séparation hydrocarbures/éther.

Par ailleurs, la récupération de l'alcool de l'effluent élimine l'étape de lavage à l'eau de l'alcool et l'étape de récupération par strippage pratiqué selon l'art antérieur. De plus, dans le cas de l'utilisation de l'éthanol pour produire de l'ETBE ou de l'ETAE, on obtient de l'éthanol exempt d'eau en vue de son recyclage dans le réacteur de synthèse d'éthers. Il est en effet connu que dans les opérations de lavage à l'eau de l'éthanol on ne peut pas obtenir, par distillation des eaux de lavage, un éthanol débarrassé d'eau, en raison des limitations azéotropiques. La présence d'eau dans le réacteur conduit à des sous-produits comme l'alcool butylique tertiaire qui sont gênants.

On réalise par conséquent des économies tant dans les équipements que dans les utilités (consommation réduite d'eau déminéralisée).

Avec un solvant préféré tel que le diméthylformamide, l'alcool extrait agit comme un solvant abaissant le point d'ébullition du mélange et permettant à la fraction oléfinique d'être distillée à une pression égale à la tension de vapeur de la fraction oléfinique à une température égale ou sensiblement inférieure à la température ambiante, sans excéder les limites de température de dégradation du solvant. A cette fin, la teneur en alcool comme co-solvant peut en effet être ajustée de manière à ce que, en fond de colonne, on se situe par exemple dans l'intervalle 0,1 à 50 % en poids, de préférence de 0,2 à 5 % en poids.

Selon une caractéristique du procédé, on peut abaisser et contrôler la température de fond de la seconde colonne (C_{III}) de distillation extractive en y introduisant de l'alcool frais à un niveau inférieur à celui de l'introduction de la fraction de fond provenant de la première colonne de distillation extractive, par exemple à un plateau situé dans le dernier tiers de la colonne ou même dans le bouilleur de la colonne. L'alcool peut être introduit selon un rapport alcool sur fraction de fond compris entre 0,001 et 1,0.

Selon une autre caractéristique du procédé, on peut recycler une partie de l'alcool provenant de l'étape de séparation, par strippage par exemple, dans la deuxième colonne de distillation extractive (C_{III}) sensiblement au même niveau que celui décrit ci-avant ou même dans le bouilleur de manière à obtenir les rapports alcool sur fraction de fond compris entre 0,001 et 1,0.

On peut même selon une autre caractéristique y introduire de l'alcool frais et de l'alcool provenant de l'étape de séparation selon ces mêmes conditions.

Selon une autre caractéristique du procédé, on peut recycler, dans la seconde zone de distillation extractive (C_{III}), la partie restante du solvant non introduit dans la colonne (C_{II}). Ce solvant peut être introduit sur un plateau supérieur à celui de l'alimentation de la colonne C_{(III)} en fraction de fond provenant de la colonne (C_{II}). Ce recyclage permet de minimiser la quantité d'alcool dans le distillat de la colonne C_{(III)}.

Selon une autre caractéristique du procédé, quand il s'agit d'un mélange d'hydrocarbures provenant d'un coupe C₄, une partie de la fraction de tête refroidie et condensée en provenance de la première colonne de distillation extractive (C_{II}) et contenant des hydrocarbures saturés peut être utilisée pour refroidir le distillat sortant de la seconde colonne de distillation extractive (C_{III}). En effet, cette fraction de tête peut être vaporisée dans la calandre d'un réfrigérant approprié. Elle est ensuite condensée et réintroduite comme reflux dans la colonne C_{(II)}. Le distillat ainsi refroidi à l'intérieur de l'échangeur est condensé et une partie de ce distillat condensé peut être réintroduite comme reflux dans la colonne (C_{III}), tandis que la partie restante peut être envoyée à l'alimentation du réacteur d'isomérisation squelettale.

Les conditions opératoires dans le réacteur de synthèse de MTBE, d'ETBE, de TAME ou d'ETAE et dans la colonne de distillation (C_{I}) sont connues de l'homme de l'art. Elles sont décrites par exemple dans les brevets FR 2440931, US4267393.

Il en est de même pour le réacteur d'isomérisation squelettale dont les conditions opératoires sont mentionnées dans les brevets US3584070 et US3696163 et dans la demande de brevet français FR 2 695 636.

Par contre, les paramètres structurels et opératoires de la première colonne de distillation extractive (C_{II}) sont en général les suivants :

Efficacité globale (en plateaux théoriques) : supérieure à 20, de préférence 40 à 50.

Pression : 1 à 25 bar ; de préférence : 4 à 15 bar pour une coupe C₄ et 2 à 12 bar pour une coupe C₅.

Rapport solvant sur charge : 3 à 10, de préférence : 4 à 6.

Taux de reflux : supérieur à 0,1, par exemple 0,15 à 15, de préférence : 1 à 6.

Les conditions dans la seconde zone de distillation extractive (C_{III}) sont en général les suivantes :

Efficacité globale (en plateaux théoriques) : supérieure à 10, de préférence 25 à 35.

Pression : 1 à 15 bar ; de préférence : 1,5 à 5 bar.

Rapport solvant sur charge : 0,01 à 0,5, de préférence : 0,02 à 0,1

Taux de reflux : supérieur à 0,1, par exemple 0,1 à 15, de préférence : 0,2 à 1.

Les conditions dans la colonne de séparation (C_{IV}) dépendent de la nature du solvant de distillation extractive et sont habituellement les suivantes :

Efficacité globale (en plateaux théoriques) : supérieure à 5, de préférence 10 à 35.

Pression : 0,1 à 3 bar ; de préférence : 0,6 à 1 bar.

Taux de reflux : supérieur à 0,2, de préférence : 1,5 à 3,5.

On peut utiliser des colonnes de distillation conventionnelles ou des colonnes garnies, connues de l'homme du métier.

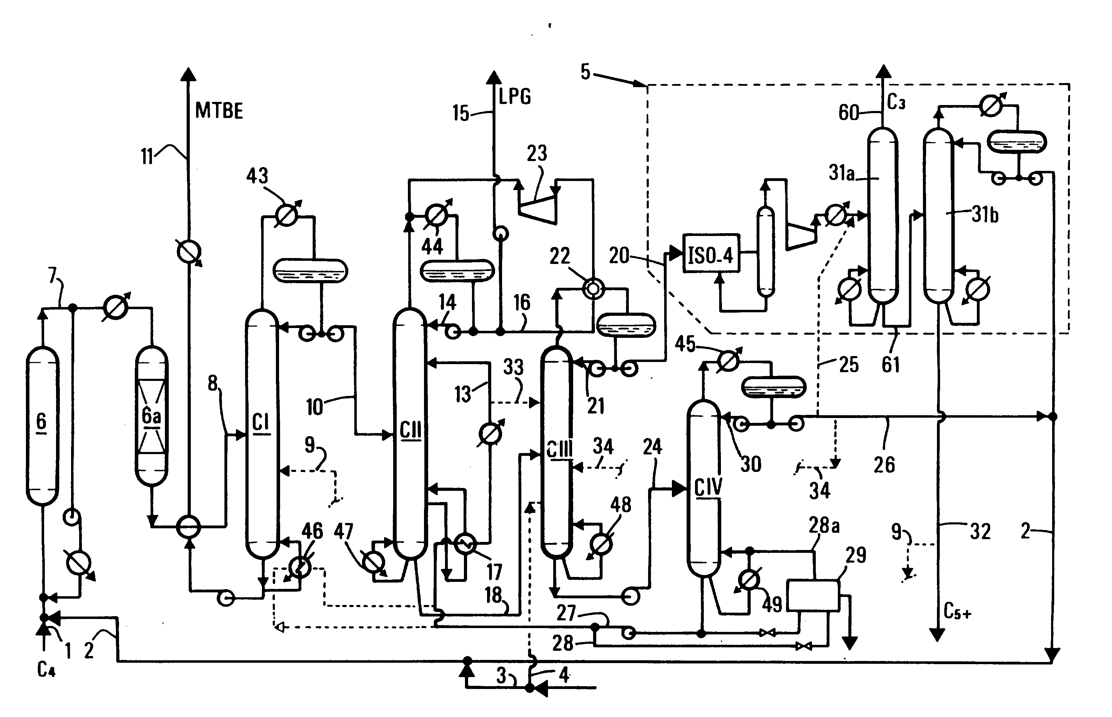

Le procédé selon l'invention est illustré par la figure jointe qui décrit de manière schématique et à titre d'exemple la synthèse du MTBE à partir d'isobutène provenant d'une coupe C4 de craquage catalytique et de méthanol. Il est bien entendu que la synthèse d'ETBE peut être réalisée selon ce même schéma, à partir d'isobutène et d'éthanol.

Un mélange d'hydrocarbures provenant d'une coupe C4 est introduit par une ligne 1 dans un réacteur 6 catalytique de synthèse de MTBE, à laquelle sont connectées une ligne 2 apportant du méthanol recyclé et des oléfines en C4 provenant d'une unité d'isomérisation 5, et une ligne 3 alimentant au moins en sortie le réacteur 6 en éthanol frais. Ce réacteur contient des billes de résine acide (Amberlyst 15 par exemple, (Rohm et Haas) sous forme d'un lit expansé). La réaction exothermique de synthèse conduit à la production d'environ au moins 75 % d'éther. Dans ces conditions, le réacteur primaire 6 peut être suivi d'un réacteur secondaire 6a qui reçoit par une ligne 7 l'effluent du réacteur 6 et qui fonctionne en général à plus basse température. Le taux de conversion d'isobutène peut alors atteindre 97 % mais ce niveau de conversion n'est absolument pas nécessaire dans le cadre de la présente invention. Il est souhaitable d'opérer la réaction avec un excès de méthanol pour améliorer la cinétique de cette réaction. Cet excès de méthanol est en général équivalent à la quantité de méthanol azéotropique laissé dans l'effluent de la réaction de synthèse contenant les hydrocarbures de la coupe C4 et qui est ensuite recyclé dans le réacteur 6 de synthèse après séparation des hydrocarbures saturés.

L'effluent de réaction 8 sortant du réacteur secondaire 6a et contenant MTBE, l'isobutène non converti, le méthanol en excès non converti, d'autres sous-produits et les hydrocarbures de la coupe C4, qui n'ont pas réagi sont introduits dans une colonne (C_{I}) de distillation conventionnelle ou catalytique, à un niveau généralement situé près du centre de la colonne. On recueille en tête un distillat par une ligne 10 qui contient les constituants de point d'ébullition inférieur à celui du MTBE et on récupère par une ligne 11 un résidu consistant essentiellement en MTBE à haute pureté, qui peut être mélangé à l'essence ou stocké. Le méthanol, en raison de l'azéotrope qui s'est formé avec les hydrocarbures de la coupe C4 mentionnés ci-dessus sous une pression de colonne (C_{I}) par exemple de 9 à 10 bar absolus (1 bar= 10⁵ Pa) et qui est normalement un constituant à plus haut point d'ébullition que le MTBE, se retrouve dans le distillat et donc dans la ligne 10. En raison du reflux assuré en tête de colonne, la teneur en MTBE du distillat est maintenue par exemple en dessous de 100 ppm en mole.

Le distillat, de préférence condensé par un échangeur de chaleur 43, et contenant le méthanol azéotropique est introduit à sa température de bulle dans une première colonne (C_{II}) de distillation extractive équivalente à environ 50 plateaux théoriques opérant à 6,5 bar absolus, à un niveau situé sensiblement près du centre de la colonne. Dans la partie supérieure de la colonne, par exemple au niveau du 6ème plateau théorique, on introduit par une ligne 13 un courant en phase liquide d'un solvant non aqueux sélectif des hydrocarbures oléfiniques, comme la N-N diméthylformamide (DMF), à une température d'environ 50°C.

Un rapport solvant sur charge d'environ 5:1 est avantageusement maintenu pour assurer la récupération dans la fraction de fond de distillation extractive d'au moins 92 % en poids des oléfines de la charge.

Le distillat contenant essentiellement des hydrocarbures saturés ne comprend généralement pas plus de 5 % de composés oléfiniques. Ce distillat est habituellement condensé sous 6 bar absolus par un dispositif d'échange thermique conventionnel 44 à température ambiante et une partie du condensat est recyclée comme reflux par une ligne 14 tandis qu'une autre partie est récupérée par une ligne 15, pour être éventuellement stockée ou envoyée à une unité d'alkylation. Il est également possible de soutirer de la ligne 15 une partie du courant de distillat (ligne 16) qui peut être utilisé en tant que réfrigérant comme on le verra ci-après.

La teneur en DMF du distillat est habituellement maintenue en dessous de 15 ppm en poids grâce à un rapport reflux sur distillat d'environ 4,0. La partie inférieure de la première colonne de distillation extractive est mise en ébullition par de la vapeur à un niveau de température approprié. Une partie de la chaleur nécessaire est fournie par échange thermique avec le solvant chaud, dans le rebouilleur 17. La fraction de fond de distillation extractive contenant les oléfines, le méthanol et le solvant est envoyée à une température de 112°C par une ligne 18 au niveau du 15ème plateau théorique d'une deuxième colonne de distillation extractive (C_{III}), équivalente à 45 plateaux théoriques. Cette colonne est opérée à une pression à laquelle, en présence de méthanol comme co-solvant, la température en fond de colonne, n'excède pas de préférence 160°C. En ajustant la teneur en méthanol à une concentration supérieure à celle de l'azéotrope on peut travailler dans des domaines de pression plus élevés. Dans ces conditions, la condensation du reflux en hydrocarbures oléfiniques en C4 en tête de colonne peut être réalisée à une température plus élevée avoisinant la température ambiante. Il est alors avantageux d'abaisser la température du mélange DMF-méthanol en introduisant du méthanol frais dans la partie inférieure de la colonne C_{III}, par la ligne 4, et/ou en introduisant du méthanol par une ligne 34 aboutissant par exemple sur le même plateau que celui de la ligne 4 et provenant d'une étape de strippage décrite ci-après.

Par exemple, la pression opératoire dans la colonne (C_{III}) est telle que le distillat oléfinique en C4 peut être totalement condensé à un point de bulle d'environ 10°C, ce qui est équivalent à une pression de 1,5 bar absolu. Si l'on introduit uniquement le méthanol azéotropique avec le solvant DMF dans la colonne C_{III}, la température du fond de colonne peut atteindre 200°C par exemple sous une pression de fond de colonne de 2,0 bar absolus. Ainsi pour limiter la température à environ 160°C par exemple, on doit rajouter dans le fond de la colonne une quantité substantielle de méthanol frais comme co-solvant par la ligne 4.

Le rapport reflux (ligne 21) sur distillat est maintenu par exemple à une valeur de 0,30, ce qui assure une teneur en DMF dans le distillat (ligne 20) riche en oléfines, d'au plus 12 ppm en poids. Selon la variante décrite ci-dessus consistant à rajouter au méthanol azéotropique une partie de l'effluent (ligne 34) de strippage contenant du méthanol on peut opérer sous une pression dans la colonne (C_{III}) de 4 bar par exemple sans excéder une température en fond de colonne de 160°C. Dans un tel schéma, le distillat peut être condensé à des températures de 30 à 40°C.

Afin de condenser le distillat de la seconde distillation extractive à 10°C par exemple une partie de la fraction de tête condensée (ligne 16) provenant de la colonne (C_{II}) riche en isobutanes est utilisé comme réfrigérant interne dans un réfrigérant (chiller) 22 où elle est vaporisée au niveau de la calandre sous une pression légèrement supérieure à la pression atmosphérique et à 0°C. Un compresseur 23 mis en mouvement par un moteur électrique comprime le courant d'isobutanes vaporisés à une pression qui est celle de la première colonne de distillation extractive C_{II} et permet ainsi la condensation de ce courant de réfrigération dans le condenseur 44. De cette façon, le procédé selon l'invention ne nécessite qu'un seul compresseur pour condenser les vapeurs de tête de la seconde distillation extractive.

Une ligne 33 raccordée à la ligne 27 d'introduction de DMF dans la colonne C_{II} peut être connectée au niveau du 6ème plateau théorique de la colonne C_{III}. On évite, de ce fait, que du méthanol ne soit entraîné vers le réacteur d'isomérisation.

Le résidu de distillation extractive est pompé (ligne 24) vers une colonne C_{IV} de strippage d'une efficacité équivalente à 30 plateaux théoriques et fonctionnant sensiblement à la pression atmosphérique pour éviter de dépasser par exemple la température de 160°C en fond de colonne.

La charge de cette colonne est introduite au niveau du 19ème plateau théorique et est distillée de façon à délivrer du méthanol en tête de colonne (ligne 26) qui une fois condensé est recyclé par la ligne 2 vers le réacteur 6 de synthèse et un résidu de strippage contenant le solvant DMF qui est recyclé par la ligne 27 vers la colonne C_{II} après avoir été refroidi à 50°C.

En effet, on peut refroidir ce solvant en prélevant une partie d'un mélange liquide dans le dernier tiers (côté bouilleur), de la première colonne (C_{II}) de distillation extractive, dont la température est inférieure de 10 à 40°C à celle du fond de cette colonne ; on vaporise alors une partie de ce mélange liquide par échange thermique dans l'échangeur 17 avec le solvant chaud provenant de l'étape de recyclage et on réintroduit le mélange liquide partiellement vaporisé sensiblement au même endroit. On peut aussi refroidir ce solvant à recycler en l'utilisant en partie comme fluide chaud dans un rebouilleur 46 en fond de colonne (C_{I}). Ce solvant peut être débarrassé de manière continue ou intermittente des composés les plus lourds et des produits de dégradation dans un régénérateur 29 connecté à la ligne de recyclage 27 par une ligne 28 et par une ligne de sortie 28a raccordée au fond de colonne (C_{IV}).

Pour assurer une bonne qualité de méthanol, on réalise de manière conventionnelle un reflux 30 en tête de la colonne de strippage (C_{IV}) que l'on maintient à un rapport de 3.0 à 1 par rapport au distillat (ligne 26).

De la même façon, ce reflux permet d'abaisser la teneur en DMF en dessous de 10 ppm, ce qui évite l'empoisonnement du catalyseur dans le réacteur de synthèse.

Selon une autre variante, si la teneur en DMF dans le distillat de la colonne de strippage qui contient le méthanol est trop élevée pour le recycler directement au réacteur, on peut envoyer par une ligne 25 raccordée à la ligne 26 une partie au moins du distillat dans un dépropaniseur 31a en aval d'une unité 5 d'isomérisation squelettale, pour le purifier.

La fraction contenant le DMF passe ensuite dans un débutaniseur 31b et le DMF est récupéré dans le résidu (ligne 32) contenant également les sous-produits C₅+. Ce résidu peut être mélangé avec l'essence, le DMF à des concentrations de l'ordre de quelques parties par million étant un bon additif pour l'essence.

Une partie au moins de ce résidu peut être recyclée par une ligne 9 raccordée à la ligne 32, à la colonne C_{I} de distillation.

Enfin, une partie du distillat (ligne 20) de la colonne (C_{III}) après avoir été condensée, est introduite dans l'unité 5 d'isomérisation squelettale qui comprend de manière conventionnelle un réacteur d'isomérisation raccordé à un dépropaniseur 31a, lequel est connecté à un débutaniseur 31b. La fraction de tête du dépropaniseur (ligne 60) contient les hydrocarbures en C3 qui sont recueillis. La fraction résiduelle contenant les hydrocarbures oléfiniques isomérisés en C4, des hydrocarbures en C5+, éventuellement du méthanol et des traces de DMF comme il a été indiqué ci-avant, est introduite par une ligne 61 dans le débutaniseur (31b). Une fraction de tête contenant les oléfines en C4 isomérisés et éventuellement du méthanol est condensée, une partie du condensat est utilisée comme reflux, tandis qu'une autre partie est recyclée par la ligne 2 vers le réacteur 6 de synthèse de MTBE.

Il est à signaler que les distillats des colonnes C_{I}, C_{II} et C_{IV} récupérés en phase gazeuse traversent respectivement des échangeurs thermiques 43, 44 et 45 pour être condensés, une partie du condensat étant utilisée comme reflux. De même, en fond des colonnes C_{I}, C_{II}, C_{III} et C_{IV}, une partie des résidus obtenus est vaporisée respectivement dans des échangeurs thermiques 46, 47, 48 et 49 et réintroduite dans les colonnes. Ces informations relatives aux reflux sont des données classiques de l'homme du métier.

Le dispositif ci-dessus sera identique pour l'éthérification d'une coupe C₅, sauf que l'utilisation de la fraction de tête condensée provenant de la colonne (CII) ne peut être envisagée comme réfrigérant interne et que le dépropaniseur et le débutaniseur seront remplacés respectivement par un débutaniseur auquel on peut éventuellement adjoindre un dépropaniseur, et un dépentaniseur.

### Exemple :

Un craqueur catalytique en lit fluidisé (FCC) délivre notamment 40180 kg/h d'un effluent sec constitué par une coupe C4 dont la composition est déduite du tableau récapitulatif ci-après.

Le procédé conventionnel de synthèse de MTBE avec deux réacteurs 6 et 6a convertit jusqu'à 97 % d'isobutène contenu dans la charge, qui a réagi avec 123 kmoles/h de méthanol frais introduit par la ligne 3. Un courant de recyclage 2 ou 26 en quantité équivalente à la quantité de méthanol qui est strippé par azéotropie avec le distillat de la colonne C_{I} est rajouté au méthanol frais, de sorte que la quantité totale de méthanol représente 154 kmoles/h.

123 kmoles/h de méthanol réagissent avec la charge fraîche d'isobutènes pour donner 123 kmoles/h de MTBE qui sont récupérés comme résidu dans la colonne C_{I} de distillation qui opère à 11 bar absolus. 31 kmoles/h de méthanol se retrouvent sous forme azéotropique dans le distillat (ligne 10) avec 580 kmoles/h de coupe C4 et d'isobutène non converti, soit une teneur en méthanol d'environ 5 % en moles dans le distillat total. Ce distillat 10 constituant la charge de la première colonne C_{II} de distillation extractive a une composition qui peut être déduite du tableau récapitulatif ci-après.

166 000 kg/h de diméthylformamide (DMF) recyclé sont introduits à 50°C au niveau du 6ème plateau théorique de la colonne C_{II}. On récupère, comme résidu (ligne 18), à une température de 112°C et sous une pression de 7,0 bar absolus tout le DMF et le méthanol, environ 10 % des hydrocarbures saturés en C4 et plus de 95 % d'oléfines en C4.

Le distillat recueilli en ligne 15 ou 16 a la composition présentée dans le tableau récapitulatif. Le résidu obtenu en ligne 18 qui contient 20 645 kg/h de méthanol et d'hydrocarbures en C4 oléfiniques dans 166 000 kg/h de DMF est envoyé sous pression dans la seconde colonne C_{III} de distillation extractive opérant à une pression en tête de 2,0 bar absolus. Un rajout de DMF (ligne 33) correspondant à 4600 kg/h est introduit au niveau du 6ème plateau théorique de cette colonne C_{III}. Cette colonne délivre un distillat par la ligne 20 dont la composition peut être déduite du tableau ci-après. Ce distillat est introduit dans le réacteur d'isomérisation squelettale.

Le résidu de colonne C_{III} (ligne 24) est envoyé dans une colonne de strippage C_{IV} d'une efficacité équivalente à 30 plateaux théoriques.

Ce résidu contient du méthanol comme co-solvant, à une concentration qui peut varier d'un minimum qui correspond à la concentration azéotropique avec les hydrocarbures de la colonne C_{I} jusqu'à un maximum correspondant aux rajouts par les lignes 4 et 34. Dans le présent exemple, la teneur en méthanol correspond à la composition azéotropique à laquelle ont été rajoutés 4000 kg/h de méthanol.

La charge de cette colonne de strippage C_{IV} est introduit au niveau du 19ème plateau théorique. La colonne fonctionne juste au-dessus de la pression atmosphérique, ce qui permet de condenser le méthanol par refroidissement indirect à l'air à environ 45°C. Une partie du méthanol condensé est réintroduit comme reflux selon un rapport de 2,6:1. Dans ces conditions, il ne reste que moins de 10,0 ppm en poids de DMF dans le méthanol. Celui-ci est recyclé avec l'effluent provenant du réacteur d'isomérisation, dans le réacteur de synthèse de MTBE.

La fraction de fond de la colonne de strippage (ligne 27) contient le solvant avec 0,002 à 0,5 % poids de méthanol et de composés plus légers.

Le bilan matière est présenté dans le tableau ci-après :

| Constituants | Effluents FCC | Charge C_{II} | Distillat C_{II} | Distillat C_{III} | Méthanol * | Distillat C_{IV} |
|---|---|---|---|---|---|---|
| C3 | 22,67 | 22,67 | 22,67 | - | - | - |
| Isobutane | 179,94 | 179,94 | 179,90 | 0,04 | - | - |
| Isobutène | 125,07 | 1,24 | 0,64 | 0,60 | - | - |
| 1-butene | 103,86 | 103,78 | 11,95 | 91,83 | - | - |
| N-butane | 54,28 | 54,28 | 38,27 | 16,01 | - | - |
| 2 Trans Butene | 120,13 | 120,04 | 0,08 | 120,25 | - | 0,07 |
| 2 Cis Butène | 96,78 | 96,74 | - | 96,64 | - | 0,10 |
| C5+ | 7,64 | 1,09 | - | 1,00 | - | 0,09 |
| Méthanol | | 31,00 | - | - | 125,0 | 156,0 |
| DME | | 0,22 | - | 0,10 | - | 0,12 |
| DMF | - | - | - | - | - | - |
| | | | | | | |
| Total (kmoles/h) | 710,37 | 611,0 | 253,51 | 334,28 | 125,0 | 156,38 |
| Masse molaire | 56,56 | 55,89 | 56,76 | 56,26 | | 33,50 |
| kg/h | 40 180 | 34 150 | 14 370 | 18 500 | 4 000 | 5 280 |

| | | | | | | |
|---|---|---|---|---|---|---|
| * Méthanol introduit dans la seconde distillation extractive | | | | | | |

## Revendications

1. Procédé de production d'un éther tertiaire dans lequel on réalise les étapes suivantes :
a) On fait réagir dans des conditions de synthèse appropriées une charge hydrocarbonée contenant des hydrocarbures saturés et des hydrocarbures isooléfiniques tertiaires avec un alcool en excès qui est le méthanol si l'on produit du méthyltertiobutyléther (MTBE) ou d'un méthytertioamylether (TAME), ou l'éthanol si l'on produit de l'éthyltertiobutyléther (ETBE) ou de l'éthyltertioamylether (ETAE), dans au moins une zone (6) de réaction catalytique et on produit un effluent contenant l'éther tertiaire;
b) On distille l'effluent dans une zone de distillation (CI) dans des conditions de distillation adéquates et on récupère, en fond de zone de distillation l'éther tertiaire, et en tête un distillat comprenant des hydrocarbures oléfiniques, une partie au moins dudit alcool et les hydrocarbures saturés, que, éventuellement l'on condense au moins en partie ;
c) On réalise dans une première zone de distillation extractive (C_{II}), dans des conditions de première distillation extractive appropriée, une distillation extractive du distillat de l'étape b), en présence d'un solvant non aqueux sélectif des hydrocarbures oléfiniques qui est introduit au moins en partie dans ladite première zone et on récupère au moins en partie une fraction de tête riche en au moins une partie des hydrocarbures saturés que l'on condense au moins en partie et une fraction de fond de première distillation extractive;
d) On réalise dans une seconde zone de distillation extractive (C_{III}), dans des conditions de seconde distillation extractive adéquates une distillation extractive de la fraction de fond de l'étape c) et l'on récupère en tête au moins en partie un distillat de seconde distillation extractive riche en hydrocarbures oléfiniques que l'on condense au moins en partie et en fond, un résidu contenant essentiellement de l'alcool et du solvant;
e) On effectue une étape de séparation du résidu de l'étape d) dans une zone de séparation (C_{IV}), dans des conditions de séparation appropriées et l'on récupère en tête, une partie au moins de l'alcool que l'on recycle au moins en partie à l'étape a) et en fond, du solvant que l'on recycle au moins en partie à l'étape c); et
f) On réalise une isomérisation squelettale du distillat de l'étape d) dans une zone (5) d'isomérisation squelettale comprenant au moins une zone de séparation des effluents d'isomérisation, dans des conditions d'isomérisation squelettale appropriées et l'on récupère un premier distillat d'isomérisation contenant des hydrocarbures légers en provenance de la zone de séparation et un deuxième distillat d'isomérisation contenant les hydrocarbures isooléfiniques tertiaires que l'on recycle au moins en partie à l'étape a).

2. Procédé selon la revendication 1 dans lequel du méthanol frais dans le cas de la production de MTBE ou TAME ou de l'éthanol frais s'il s'agit d'ETBE ou d'ETAE est introduit dans la seconde zone de distillation extractive (C_{III}) en un niveau inférieur au niveau d'introduction de la fraction de fond provenant de l'étape c), selon un rapport alcool sur fraction de fond compris entre 0,001 et 1,0.

3. Procédé selon les revendications 1 et 2 dans lequel une partie de l'alcool provenant de l'étape e) est recyclé dans la seconde zone de distillation extractive (C_{III}) sensiblement au même niveau que celui de l'introduction de la fraction de fond de l'étape c), selon un rapport alcool sur fraction de fond compris entre 0,001 et 1,0.

4. Procédé selon l'une des revendications 1 à 3, dans lequel la charge est une coupe C₅.

5. Procédé selon l'une des revendications 1 à 3 dans lequel la charge est une coupe C₄.

6. Procédé selon la revendication 5 dans lequel une partie de la fraction de tête condensée selon l'étape c) est vaporisée dans la calandre d'un échangeur thermique (22) de façon à condenser le distillat de seconde distillation extractive.

7. Procédé selon l'une des revendications 1 à 6, dans lequel on recycle dans la seconde zone de distillation extractive (C_{III}) sur un plateau supérieur à celui de l'alimentation en fraction de fond de l'étape c), la partie restante du solvant, non introduite à l'étape c).

8. Procédé selon l'une des revendications 1 à 7, dans lequel on purifie une partie au moins de l'alcool à recycler à l'étape a) dans la zone de séparation des effluents d'isomérisation squelettale.

9. Procédé selon l'une des revendications 1 à 8 dans lequel on récupère en fond de la zone de séparation des effluents d'isomérisation un résidu contenant les hydrocarbures C5+ ou C6+ que l'on recycle au moins en partie à la zone de distillation C_{(I)} en un plateau inférieur à celui de l'alimentation de la zone C_{(I)} en effluent de la zone de réaction catalytique.

10. Procédé selon l'une des revendications 1 à 9 dans lequel les conditions dans la zone de distillation extractive (C_{II}) sont les suivantes :
Efficacité globale (en plateaux théoriques) : supérieure à 20, de préférence 40 à 50.
Pression : 1 à 25 bar.
Rapport solvant sur charge : 3 à 10, de préférence : 4 à 6.
Taux de reflux : supérieur à 0,1, de préférence 1 à 6.

11. Procédé selon l'une des revendications 1 à 10 dans lequel les conditions dans la seconde zone de distillation extractive (C_{III}) sont les suivants :
Efficacité globale (en plateaux théoriques) : supérieure à 10, de préférence 25 à 35.
Pression : 1 à 15 bar.
Rapport solvant sur charge : 0,01 à 0,5, de préférence : 0,02 à 0,1.
Taux de reflux : supérieur à 0,1, de préférence 0,2 à 1.

12. Procédé selon l'une des revendications 1 à 11 dans lequel les conditions dans la zone de séparation (C_{IV}) sont les suivantes :
Efficacité globale (en plateaux théoriques) : supérieure à 5, de préférence 10 à 35.
Pression : 0,1 à 3 bar ; de préférence : 0,6 à 1 bar.
Taux de reflux : supérieur à 0,2, de préférence 1,5 à 3,5.

13. Procédé selon l'une des revendications 1 à 12 dans lequel on prélève une partie d'un mélange liquide dans le derniers tiers de la première colonne (C_{II}) de distillation extractive, ayant une température de 10 à 40°C inférieure à celle du fond de ladite colonne, on vaporise en partie ce mélange liquide par échange thermique avec le solvant provenant de l'étape de séparation et on réintroduit le mélange liquide partiellement vaporisé sensiblement au même endroit.

14. Procédé selon l'une des revendications 1 à 13 dans lequel on réalise un rebouillage d'une partie de l'éther tertiaire par échange indirect de chaleur avec une partie du solvant provenant de l'étape de séparation.

15. Procédé selon l'une des revendications 1 à 14 dans lequel le solvant est choisi dans le groupe formé par la N-N diméthylformamide, la N-N diéthylformamide, la N-N diméthylacétamide, la formylmorpholine, la N-méthylpyrrolidone, la butyrolactone et le furfural et de préférence le solvant est la N-N diméthylformamide.

16. Procédé selon l'une des revendications 1 à 15 dans lequel on récupère en fond de seconde zone de récupération extractive (C_{III}) le résidu à une température au plus égale à 220°C (étape d).

## Patentansprüche

1. Verfahren zur Herstellung eines tertiären Ethers, bei dem man die folgenden Stufen durchführt:
a) Reagierenlassen einer Kohlenwasserstoff-Beschickung, die gesättigte Kohlenwasserstoffe und tertiäre Olefin-Kohlenwasserstoffe enthält, unter geeigneten Synthese-Bedingungen mit einem Alkohol im Überschuß, bei dem es sich um Methanol handelt, wenn man den Methyl-tert-butylether (MTBE) oder den Methyl-tert-amylether (TAME) herstellt, oder bei dem es sich um Ethanol handelt, wenn man den Ethyl-tert-butylether (ETBE) oder den Ethyl-tert-amylether (ETAE) herstellt, in mindestens einer katalytischen Reaktionszone (6) unter Bildung eines Abstroms, der den tertiären Ether enthält;
b) Destillieren des Abstroms in einer Destillationszone (C_{I}) unter geeigneten Destillations-Bedingungen und Gewinnung des tertiären Ethers am Boden der Destillationszone und eines Destillats, das Olefin-Kohlenwasserstoffe, mindestens einen Teil des genannten Alkohols und die gesättigten Kohlenwasserstoffe enthält, am Kopf der Destillationszone, das man gegebenenfalls mindestens zum Teil kondensiert;
c) Durchführung einer extraktiven Destillation mit dem Destillat aus der Stufe (b) in einer ersten extraktiven Destillationszone (C_{II}) unter geeigneten ersten extraktiven Destillations-Bedingungen in Gegenwart eines nicht-wäßrigen Lösungsmittel, das für Olefin-Kohlenwasserstoffe selektiv ist und das mindestens zum Teil in die genannte erste Zone eingeführt wird, und Gewinnung mindestens zum Teil einer Kopffraktion, die reich an mindestens einem Teil der gesättigten Kohlenwasserstoffe ist, die man mindestens zum Teil kondensiert, und einer Bodenfraktion aus der ersten extraktiven Destillation;
d) Durchführung einer extraktiven Destillation der Bodenfraktion aus der Stufe (c) in einer zweiten extraktiven Destillationszone (C_{III}) unter geeigneten zweiten extraktiven Destillations-Bedingungen und Gewinnung mindestens zum Teil eines zweiten extraktiven Destillationsdestillats am Kopf, das reich an Olefin-Kohlenwasserstoffen ist, das man mindestens zum Teil kondensiert, und eines Rückstands am Boden, der im wesentlichen den Alkohol und das Lösungsmittel enthält;
e) Durchführung einer Stufe zur Abtrennung des Rückstandes aus der Stufe (d) in einer Trennzone (C_{IV}) unter geeigneten Abtrennungs-Bedingungen und Gewinnung mindestens eines Teils des Alkohols am Kopf, den man mindestens zum Teil in die Stufe (a) recyclisiert, und des Lösungsmittels am Boden, das man mindestens zum Teil in die Stufe (c) recyclisiert; und
f) Durchführung einer Skelett-lsomerisierung mit dem Destillat aus der Stufe (d) in einer Skelett-lsomerisierungszone (5), die mindestens eine Zone zur Abtrennung der Isomerisierungs-Abströme umfaßt, unter geeigneten Skelett-lsomerisierungs-Bedingungen und Gewinnung eines ersten Isomerisierungs-Destillats, das leichte Kohlenwasserstoffe enthält, die aus der Trennzone stammen, und eines zweiten Isomerisierungs-Destillats, das die tertiären Isoolefin-Kohlenwasserstoffe enthält, die man mindestens zum Teil in die Stufe (a) recyclisiert.

2. Verfahren nach Anspruch 1, bei dem man frisches Methanol, für den Fall, daß MTBE oder TAME hergestellt wird, oder frisches Ethanol, für den Fall, daß ETBE oder ETAE hergestellt wird, in die zweite extraktive Destillationszone (C_{III}) in einem Niveau unterhalb des Niveaus der Einführung der Bodenfraktion aus der Stufe (c) in einem Verhältnis von Alkohol zu Bodenfraktion zwischen 0,001 und 1,0 einführt.

3. Verfahren nach den Ansprüchen 1 und 2, bei dem man einen Teil des Alkohols, der aus der Stufe (e) stammt, in die zweite extraktive Destillationszone (C_{III}) im wesentlichen auf dem gleichen Niveau wie die Einführung der Bodenfraktion aus der Stufe (c) in einem Verhältnis von Alkohol zu Bodenfraktion zwischen 0,001 und 1,0 recyclisiert.

4. Verfahren nach den Ansprüchen 1 bis 3, bei dem die Beschickung eine C₅-Fraktion (C₅-Schnitt) ist.

5. Verfahren nach den Ansprüchen 1 bis 3, bei dem die Beschickung eine C₄-Fraktion (C₄-Schnitt) ist.

6. Verfahren nach Anspruch 5, bei dem ein Teil der in der Stufe (c) kondensierten Kopffraktion in dem Kalander eines Wärmeaustauschers (22) so verdampft wird, daß das Destillat der extraktiven zweiten Destillation kondensiert wird.

7. Verfahren nach einem der Ansprüche 1 bis 6, bei dem man den restlichen Teil des Lösungsmittels, der nicht in die Stufe (c) eingeführt worden ist, in die zweite extraktive Destillationszone (C_{III}) auf einen Boden recyclisiert, der höher ist als derjenige der Zuführung der Bodenfraktion aus der Stufe (c).

8. Verfahren nach einem der Ansprüche 1 bis 7, bei dem man mindestens einen Teil des in die Stufe (a) zu recyclisierenden Alkohols in der Zone zur Abtrennung der Skelettisomerisierungs-Abströme reinigt.

9. Verfahren nach einem der Ansprüche 1 bis 8, bei dem man am Boden der Zone zur Abtrennung der Isomerisierungs-Abströme einen Rückstand gewinnt, der C₅+- oder C₆+-Kohlenwasserstoffe enthält, den man mindestens zum Teil in die Destillationszone (C_{I}) recyclisiert in einen Boden, der unterhalb desjenigen liegt, dem man in der Zone (C_{I}) den Abstrom aus der katalytischen Reaktionszone zuführt.

10. Verfahren nach einem der Ansprüche 1 bis 9, bei dem die Bedingungen in der extraktiven Destillationszone (C_{II}) die folgenden sind:
Gesamtwirkungsgrad (ausgedrückt in theoretischen Böden):> 20, vorzugsweise 40 bis 50,
Druck: 1 bis 25 bar,
Verhältnis Lösungsmittel/Beschickung: 3 bis 10, vorzugsweise 4 bis 6, Rückfluß-Verhältnis: > 0,1, vorzugsweise bis 6.

11. Verfahren nach einem der Ansprüche 1 bis 10, bei dem die Bedingungen in der zweiten extraktiven Destillationszone (C_{III}) die folgenden sind:
Gesamtwirkungsgrad (ausgedrückt in theoretischen Böden): > 10, vorzugsweise 25 bis 35,
Druck: 1 bis 15 bar,
Verhältnis Lösungsmittel/Beschickung: 0,01 bis 0,5, vorzugsweise 0,02 bis 0,1, Rückfluß-Verhältnis: > 0,1, vorzugsweise 0,2 bis 1.

12. Verfahren nach einem der Ansprüche 1 bis 11, bei dem die Bedingungen in der Trennzone (C_{IV}) die folgenden sind:
Gesamtwirkungsgrad (ausgedrückt in theoretischen Böden): > 5, vorzugsweise 10 bis 35,
Druck: 0,1 bis 3 bar, vorzugsweise 0,6 bis 1 bar,
Rückfluß-Verhältnis: > 0,2, vorzugsweise 1,5 bis 3,5.

13. Verfahren nach einem der Ansprüche 1 bis 12, bei dem man einen Teil einer flüssigen Mischung im letzten Drittel der ersten extraktiven Destillationskolonne (C_{II}) entnimmt, die eine Temperatur hat, die um 10 bis 40°C unterhalb derjenigen des Bodens der genannten Kolonne liegt, diese flüssige Mischung durch Wärmeaustausch mit dem Lösungsmittel, das aus der Trennzone stammt, zum Teil verdampft und die teilweise verdampfte flüssige Mischung im wesentlichen an der gleichen Stelle wieder in die flüssige Mischung einführt.

14. Verfahren nach einem der Ansprüche 1 bis 13, bei dem man mindestens einen Teil des tertiären Ethers durch indirekten Wärmeaustausch mit einem Teil des Lösungsmittels aus der Trennzone durch Sieden entfernt.

15. Verfahren nach einem der Ansprüche 1 bis 14, bei dem das Lösungsmittel ausgewählt wird aus der Gruppe, die besteht aus N,N-Dimethylformamid, N,N-Diethylformamid, N,N-Dimethylacetamid, Formylmorpholin, N-Methylpyrrolidon, Butyrolacton und Furfural, und bei dem das Lösungsmittel vorzugsweise N,N-Dimethylformamid ist.

16. Verfahren nach einem der Ansprüche 1 bis 15, bei dem man am Boden der zweiten extraktiven Gewinnungszone (C_{III}) den Rückstand bei einer Temperatur von höchstens gleich 220°C (Stufe d) gewinnt (abtrennt).

## Claims

1. A process for the production of a tertiary ether comprising the following steps:
a) reacting a hydrocarbon feedstock containing saturated hydrocarbons and tertiary iso-olefinic hydrocarbons with an alcohol in excess which is methanol if the product is methyltertiobutylether (MTBE) or methyltertioamylether (TAME), or ethanol if the product is ethyltertiobutylether (ETBE) or ethyltertioamylether (ETAE), in at least one catalytic reaction zone (6) to produce an effluent containing the tertiary ether,
b) distilling the effluent in a distillation zone (C_{I}) under suitable distillation conditions, the tertiary ether being recovered from the bottom of the tertiary ether distillation zone, and a distillate containing olefin hydrocarbons, at least a portion of said alcohol and saturated hydrocarbons which may be at least partially condensed, being recovered overhead,
c) carrying out extractive distillation of the distillate from step b) in a first extractive
| Constituents | Effluents | Feed | Distillate | Distillate | Methanol | Distillate |
|---|---|---|---|---|---|---|
| | FCC | C_{II} | C_{II} | C_{III} | * | C_{IV} |
| C3 | 22.67 | 22.67 | 22.67 | - | - | - |
| Isobutane | 179.94 | 179.94 | 179.90 | 0.04 | - | - |
| Isobutene | 125.07 | 1.24 | 0.64 | 0.60 | - | - |
| 1-butene | 103.86 | 103.78 | 11.95 | 91.83 | - | - |
| N-butane | 54.28 | 54.28 | 38.27 | 16.01 | - | - |
| 2-trans butene | 120.13 | 120.04 | 0.08 | 120.25 | - | 0.07 |
| 2-cis butene | 96.78 | 96.74 | - | 96.64 | - | 0.10 |
| C5+ | 7.64 | 1.09 | - | 1.00 | - | 0.09 |
| Methanol | - | 31.00 | - | - | 125.0 | 156.0 |
| DME | - | 0.22 | - | 0.10 | - | 0.12 |
| DMF | - | - | - | - | - | - |
| | | | | | | |
| Total (kmoles/h) | 710.37 | 611.0 | 253.51 | 334.28 | 125.0 | 156.38 |
| Molar mass | 56.56 | 55.89 | 56.76 | 56.26 | = | 33.50 |
| kg/h | 40 180 | 34 150 | 14 370 | 18 500 | 4 000 | 5 280 |
| | | | | | | |
|---|---|---|---|---|---|---|
| * Methanol introduced in second extractive distillation. | | | | | | |
distillation zone (C_{II}) under appropriate first extractive distillation conditions, in the presence of a non aqueous solvent which is selective for olefin hydrocarbons, at least a portion of which is introduced into said first zone and an overhead fraction being recovered which is rich in at least a portion of the saturated hydrocarbons which is at least partially condensed, a bottom fraction also being recovered from the first extractive distillation step,
d) carrying out extractive distillation of the bottom fraction from step c) in a second extractive distillation zone (C_{III}), under suitable second extractive distillation conditions, and recovering overhead at least a portion of a second extractive distillation distillate which is rich in olefin hydrocarbons and which is at least partially condensed, a residue containing mainly alcohol and solvent being recovered from the bottom,
e) separating the residue from step d) in a separation zone (C_{IV}), under appropriate separation conditions, and recovering overhead at least a portion of the alcohol and recycling it at least in part to step a), and recovering solvent from the bottom and recycling it at least in part to step c), and
f) carrying out skeletal isomerisation of the distillate from step d) in a skeletal isomerisation zone under appropriate skeletal isomerisation conditions, the zone comprising at least one separation zone for the isomerisation effluents, recovering from said separation zone a first isomerisation distillate containing light hydrocarbons from the separation zone and a second isomerisation distillate containing tertiary isoolefin hydrocarbons which is recycled to step a).

2. Process according to claim 1 wherein fresh methanol in the case of MTBE or TAME production or fresh ethanol in the case of ETBE OR ETAE production is introduced into the second extractive distillation zone (C_{III}) at a level which is lower than that where the bottom fraction from step c) is introduced, in a ratio of alcohol to bottom fraction of between 0.001 and 1.0.

3. Process according to claim 1 or claim 2 wherein a portion of the alcohol from step e) is recycled to the second extractive distillation zone (C_{III}) at substantially the same level as that for the introduction of the bottom fraction from step c), in a ratio of alcohol to bottom fraction of between 0.001 and 1.0.

4. Process according to any one of claims 1 to 3 wherein the feedstock is a C₅ fraction.

5. Process according to any one of claims 1 to 3 wherein the feedstock is a C₄ fraction.

6. Process according to claim 5 wherein a portion of the condensed overhead fraction from step c) is vaporised in the grille of a heat exchanger (22) to condense the distillate from the second extractive distillation step.

7. Process according to any one of claims 1 to 6 wherein the remaining portion of solvent not introduced at step c) is recycled to the second extractive distillation zone (C_{III}) to a plate which is above that of the supply for the bottom fraction from step c).

8. Process according to any one of claims 1 to 7 wherein at least a portion of the alcohol recycled to step a) is purified in the separation zone for the effluent from the skeletal isomerisation step.

9. Process according to any one of claims 1 to 8 wherein a residue containing C₅⁺ or C₆⁺ hydrocarbons is recovered from the bottom of the separation zone for the isomerisation effluents and recycled at least in part to the distillation zone (C_{I}) at a plate which is lower than that for the supply of the effluent from the catalytic reaction zone to zone (C_{I}).

10. Process according to any one of claims 1 to 9 wherein the conditions in the extractive distillation zone (C_{II}) are as follows:
Total efficiency (in theoretical plates): more than 20, preferably 40 to 50;
Pressure: 1 to 25 bar;
Solvent to feedstock ratio: 3 to 10, preferably 4 to 6;
Reflex rate: more than 0.1, preferably 1 to 6.

11. Process according to any one of claims 1 to 10 wherein the conditions in the second extractive distillation zone (C_{III}) are as follows:
Total efficiency (in theoretical plates): more than 10, preferably 25 to 35;
Pressure: 1 to 15 bar;
Solvent to feedstock ratio: 0.01 to 0.5,
preferably 0.02 to 0.1;
Reflux rate: more than 0.1, preferably 0.2 to 1.

12. Process according to any one of claims 1 to 11 wherein the conditions in the separation zone (C_{IV}) are as follows:
Total efficiency (in theoretical plates): more than 5, preferably 10 to 35;
Pressure: 0.1 to 3 bar, preferably 0.6 to 1 bar;
Reflux rate: more than 0.2, preferably 1.5 to 3.5.

13. Process according to any one of claims 1 to 12 wherein a portion of the liquid mixture in the last third of the first extractive distillation column (C_{II}), with a temperature 10°C to 40°C below that at the bottom of said column, is removed and partially vaporised by heat exchange with the solvent from the separation step and the partially vaporised liquid mixture is reintroduced at substantially the same point.

14. Process according to any one of claims 1 to 13 wherein a portion of the tertiary ether is reboiled by indirect heat exchange with a portion of the solvent from the separation step.

15. Process according to any one of claims 1 to 14 wherein the solvent is selected from the group formed by N,N-dimethylformamide, N,N-diethylformamide, N,N-dimethylacetamide, formylmorpholine, N-methylpyrrolidone, butyrolactone and furfural, preferably N,N-dimethylformamide.

16. Process according to any one of claims 1 to 15 wherein the residue is recovered from the bottom of the second extractive distillation zone (C_{III}) at a temperature of at most 220°C (step d)).
